(19) Europäisches Patentamt
European Patent Office
Office européen des brevets

(11) Publication number: **0 016 851**
**B1**

(12) **EUROPEAN PATENT SPECIFICATION**

(45) Date of publication of patent specification: 26.01.83

(21) Application number: 79100985.5

(22) Date of filing: 02.04.79

(51) Int. Cl.³: C 07 C 1/04, B 01 J 35/10, B 01 J 27/04, B 01 J 23/28, B 01 J 23/30 //C07C11/02, B01J31/04

(54) **Process for producing olefins from carbon monoxide and hydrogen.**

(43) Date of publication of application:
15.10.80 Bulletin 80/21

(45) Publication of the grant of the patent:
26.01.83 Bulletin 83/4

(84) Designated Contracting States:
DE FR GB

(56) References cited:
DE - C - 597 515
FR - A - 1 019 738
GB - A - 833 976
GB - A - 1 219 281
NL - C - 56 879
US - A - 2 490 488
US - A - 3 941 819
US - A - 4 042 614
US - A - 4 042 615
US - A - 4 045 461
US - A - 4 115 075

(73) Proprietor: THE DOW CHEMICAL COMPANY
Dow Center 2030 Abbott Road Post Office Box 1967
Midland Michigan 48640 (US)

(72) Inventor: Murchison, Craig Brian
606 West Meadowbrook
Midland Midland Michigan (US)
Inventor: Murdick, Dewey A.
705 East Curtis
Hope Midland Michigan (US)

(74) Representative: Casalonga, Alain et al,
BUREAU D.A. CASALONGA OFFICE JOSSE & PETIT Baaderstrasse 12-14
D-8000 München 5 (DE)

EP 0 016 851 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for producing olefins from carbon monoxide and hydrogen

This invention relates to an improved Fischer-Tropsch process for the production of olefinic $C_2$—$C_4$ hydrocarbons.

The prior art contains many examples of metals known to be useful in reacting carbon monoxide with hydrogen to produce a variety of compounds—both hydrocarbons and oxygenated compounds. These metals include, among others, Mo, W, Th, Ru, Re, Pd, Ni, Co, and Fe. It is upon the last three of these metals that most commercial experience is based. In the Fischer-Tropsch synthesis, carbon monoxide and hydrogen are reacted over an iron or cobalt catalyst to produce saturated and unsaturated hydrocarbons and oxygenated compounds containing from one to as many as one thousand carbon atoms. The hydrocarbons can be aliphatic, alicyclic, or aromatic. Commercial utilisation of this synthesis prior to 1950 was accomplished largely in Germany and is summarized in Storch, Columbic, and Anderson: *The Fischer-Tropsch and Related Synthesis,* John Wiley and Sons, New York 1951.

The major disadvantages in the prior art processes and catalysts is that most of them are not capable of selectively producing olefins. A process capable of producing olefins of low molecular weight could be used as a source of feedstock for a variety of petrochemical plants.

The present invention is a process for producing olefins $C_2$—$C_4$ hydrocarbons by contacting carbon monoxide and hydrogen at reactive conditions, characterized by contacting the carbon monoxide and hydrogen with a catalyst having a surface area less than 100 $m^2/g$ and consisting of:

(A) between 1 percent and 95 percent by weight based upon the weight of the catalyst of at least one material selected from the group consisting of the sulfide, oxide or metal of molybdenum, tungsten, rhenium, ruthenium, nickel, palladium, rhodium, osmium, iridium and platinum; and

(B) between 0.05 percent and 50 percent by weight based upon the weight of the catalyst of at least one material selected from the group consisting of the hydroxide, oxide or salt of lithium, sodium potassium, rubidium, cesium, magnesium, calcium, strontium, barium and thorium.

Optionally, the catalyst can contain as a third component a support. This invention markedly increases the production of $C_2$—$C_4$ olefins, particularly $C_3$ and $C_4$ olefins.

The carbon monoxide required for the process can be obtained from any carbon source, such as from the degradation of coal or of high molecular weight hydrocarbon residuals. The molar ratio of hydrogen to carbon monoxide ranges generally from at least about 0.25 and preferably about 0.5 to an upper limit of about 4 and preferably about 1.5.

Process reaction conditions can vary over a rather broad range. The pressure can vary from at least about 137,20x$10^2$ Pa (1 psig) and preferably about 5194x$10^2$Pa (75 psig) to an upper limit of about 103.880x$10^2$ Pa (1500 psig) and preferably about 34496x$10^2$ (500 psig). The reaction temperature ranges from at least about 200°C and preferably about 300°C to an upper limit of about 600°C and preferably about 400°C.

The catalyst is typically either a two- or a three-component system. The first component is at least one material selected from the group consisting of the sulfide, oxide or metal of each of molybdenum, tungsten, rhenium, ruthenium, palladium, rhodium, osmium, iridium and platinum. As used herein, "sulfide" includes those compounds that have oxygen and sulfur directly attached to the same metal atom, such as O—Mo—S. This first component is present in an amount, based upon the weight of the catalyst, of at least about 1 and preferably at least about 10 weight percent with an upper limit of about 95 and preferably about 50 weight percent. A preferred first component is at least one material selected from the group consisting of the sulfide, oxide or metal of molybdenum or of tungsten. These molybdenum and tungsten materials exhibit exceptionally good sulfur tolerance. An especially preferred first component is at least one material selected from the group consisting of the sulfide, oxide or metal of molybdenum.

The second component is at least one material selected from the group consisting of the hydroxide, oxide or salt of each of lithium, sodium, potassium, rubidium, cesium, magnesium, calcium, strontium, barium and thorium. The second component is present in an amount, based upon the weight of the catalyst, of at least about 0.05 and preferably at least about 1 weight percent with an upper limit of about 50 and preferably about 10 weight percent. A preferred second component is at least one material selected from the group consisting of the hydroxide, oxide or salt of each of lithium, sodium, potassium, rubidium or cesium. An especially preferred second component is at least one material selected from the group consisting of the hydroxide, oxide or salt of potassium.

Obviously, the optional support is not necessary to this invention but one is often employed for reasons of convenience. Virtually any support can be used but those most typical are the many and varied forms of alumina and carbon, silica, zirconia, zircon (a mixture of zirconia and silica), titanium dioxide, magnesia or mixtures thereof. Other suitable supports can also be used. An alumina support is preferred. Based upon the weight of the catalyst, the support can comprise between about 1 and preferably at least about 40 percent of the catalyst with an upper limit of about 98.95 percent. If a support is present, most preferably it comprises at least about 60 percent of the catalyst.

Components of the catalyst of this invention can be present *per se*, or as an integral part of one another or as a combination thereof. Illustrative of components being present as an integral part of one another, certain supports (where a support is present) contain relatively small (based on the weight of the support) amounts of alkali metal hydroxides and/or oxides.

Preferred species of the catalyst of this invention have as a first component at least one material selected from the group consisting of the sulfide, oxide or metal of molybdenum or of tungsten. More preferred species have as a first component at least one material selected from the group consisting of the sulfide, oxide or metal of molybdenum or of tungsten and as a second component at least one material selected from the group consisting of the hydroxide, oxide or salt of each of lithium, sodium, potassium, rubidium and cesium. Still more preferred species have as a first component at least one material selected from the group consisting of the sulfide, oxide or metal of molybdenum or of tungsten, as a second component at least one material selected from the group consisting of the hydroxide, oxide or salt of each of lithium, sodium, potassium, rubidium and cesium, and as a third component an alumina, silica, carbon, zirconia, zircon or magnesia support. Especially preferred species have as a first component at least one material selected from the group consisting of the sulfide, oxide or metal of molybdenum, as a second component at least one material selected from the group consisting of the hydroxide, oxide or salt of potassium, and as a third component an alumina support.

It is theorized that the efficient production of olefins by the process of this invention is related to the surface area of the catalyst. Supported or unsupported, the more efficient catalysts useful in the process of the invention possess a surface area of less than about 100 square meters per gram ($m^2/g$) measured by the Brunauer, Emmett and Teller (BET) method and preferably less than about 40 $m^2/g$. The BET method is described by R. B. Anderson, *Experimental Methods in Catalytic Research,* p. 48—66 (Academic Press, 1968).

In one embodiment of this invention, the catalyst can be represented by the formulae:

$$\underset{(I)}{Ma_xH_yMeO_3C_2O_4} \quad \text{and} \quad \underset{(II)}{MbMeO_3C_2O_4}.$$

Ma is a metal selected from the group consisting of Li, Na, K, Rb, Cs, and mixtures thereof; Mb is a metal selected from the group consisting of Mg, Ca, Sr, Ba, Th, and mixtures thereof; Me is a metal selected from the group consisting of Mo and W; and x is 1 or 2 and y is 0 or 1 with the provisos that when x is 1, y is 1 and when x is 2, y is 0. Catalysts of I are preferred to catalysts of II; catalysts wherein Ma is potassium are preferred to catalysts wherein Ma is Li, Na, Rb or Cs; and catalysts wherein Ma is potassium and Me is molybdenum are especially preferred. The catalyst species of this particular embodiment have a surface area less than about 100 $m^2/g$ (generally less than about 40 $m^2/g$) and can be used either singly or in combination with one another.

The catalyst is generally prepared by dissolving two moles of oxalic acid per mole of oxide of Me in water, adding one mole of a hydrated MaMe oxide, adjusting the pH of the resulting solution to about 6, and allowing the solution to cool. Crystals of an ammonium complex of $Ma_xH_yMeO_3C_2O_4$ or $MbMeO_3C_2O_4$ form and precipitate from solution. The crystals are then reduced with hydrogen for a suitable period, e.g., about 24 hours, at a series of temperatures commencing with a minimum temperature of about 200°C and culminating in a maximum temperature of about 600°C. After reduction, the crystals are immediately useful as a catalyst in the process of the invention as they require no third component.

The following examples illustrate the invention. All percentages are by weight unless otherwise indicated.

Apparatus and procedure:

. In preparation of a supported catalyst, supports were impregnated by a technique known as the incipient wetness technique. Water-soluble salts of active components of the catalyst and a support were chosen. A quantity of water which a catalyst support will absorb is known as its pore volume. According to the desired catalyst loading, a quantity of the soluble salts are dissolved in water approximately equal to the pore volume of the support. The support was then immersed in the water which it absorbed completely. A wet cake was formed. The wet cake was first air-dried at room temperature for sixteen to twenty-four hours. It was then placed in an oven and heated at a rate of between about 0.4°C and about 1.8°C per minute in the presence of air or nitrogen to a final temperature of between about 500°C and about 650°C. The catalyst was held at this final temperature for about six hours before being allowed to cool slowly to room temperature.

In the examples, an apparatus was utilized which included in sequential order three high-pressure gas bottles, a manifold, and a reactor equipped on the downstream side with a fine metering valve and a rotameter through a sampling manifold to a gas chromatograph. One bottle contained a mixture of hydrogen and carbon monoxide in a one to one molar ratio. The second bottle contained a mixture of hydrogen and carbon monoxide in a one to two ratio. The third bottle contained hydrogen alone. Each bottle was independently connected to the manifold. The manifold was constructed such that any of

the three bottles could be used to feed the reactor. Through the sampling manifold, the product of each reactor could be piped to the gas chromatograph for analysis.

Before each run, catalyst was loaded in the reactor to be used and heated to 350°C over a four-hour period in the presence of hydrogen. The hydrogen flow and a temperature of 350°C were maintained for sixteen more hours. Then the catalyst was raised to a final temperature over a four-hour period. The final temperature was between about 500°C and about 650°C. This final temperature was held for about two to forty-eight hours. The outlet temperature of each reactor in use was maintained by the use of a hot air stream. The reactors were then lowered to operating temperature in the presence of hydrogen. Next, feed from the high-pressure gas bottle containing hydrogen and carbon monoxide was allowed to flow through the manifold to the reactor. Pressure, flow, and temperature were adjusted to operating values. Unless otherwise indicated the operating values were for the hourly space velocity: 300 $hr^{-1}$; for the temperature: 300°C; and for the pressure: $20776 \times 10^2$ Pa 300 psig. The $H_2/CO$ ratio was one unless otherwise indicated.

Subscripts in all examples indicate the number of carbon atoms. All hydrocarbon analyses are reported in carbon mole percent in all examples. "Carbon mole percent" is defined as 100 times the moles of carbon present in a hydrocarbon fraction divided by the total moles of carbon in the product hydrocarbon. If one mole of ethylene is found in the $C_2$ fraction, this is counted as two moles of carbon. The term "product hydrocarbon" excludes any carbon dioxide produced. Unless otherwise indicated, molybdenun concentrations are reported as $MoO_3$ equivalents and potassium concentrations are reported as $K_2O$ equivalents. The surface area (support plus the first and second components) of each catalyst was less than about 40 $m^2/g$, unless indicated to the contrary.

## Example 1

In order to demonstrate the effect of increasing concentration of the second component of the catalyst, the following three runs were conducted.

The catalyst of the first run (1A) was commercially available and was composed of 10 weight percent $MoO_3$ on alumina. In run 1B, the catalyst of Run 1A was modified by the addition of 2 weight percent $K_2O$ according to the incipient wetness technique described above. In run 1C, the catalyst was prepared by dissolving 93.2 g $Al(NO_3)_3 \cdot 9H_2O$, 15.1 g $(NH_4)_6Mo_7O_{24} \cdot 4H_2O$, and 3.6 g $KNO_3$ in 850 cc of water, evaporation of the water to form a wet cake, and drying the wet cake under reduced pressure for about 12 to 24 hours. The wet cake was then calcined by heating it in the presence of air from ambient temperature to 650°C at a rate of about 1.8°C per minute and holding it at 650°C for 6.5 hours. The catalyst was reduced to particles of about 1 mm diameter of grinding. On a weight basis, the calcined catalyst was composed of 48 percent $MoO_3$, 48 percent $Al_2O_3$, and 4 percent $K_2O$.

| | Run 1A | Run 1B | Run 1C |
|---|---|---|---|
| Paraffins: | | | |
| $C_1$ | 62.3 | 42.0 | 37.6 |
| $C_2$ | 21.6 | 31.3 | 25.8 |
| $C_3$ | 9.0 | 12.7 | 13.8 |
| $C_4$ | 3.9 | 6.6 | 4.4 |
| Olefins: | | | |
| $C_2$ | 0.9 | 2.2 | 0.9 |
| $C_3$ | — | 2.7 | 8.7 |
| $C_4$ | — | 0.9 | 4.8 |
| Olefins & paraffins: | | | |
| $C_5$ | 2.3 | 1.5 | 2.1 |
| $C_6$ | — | — | 1.8 |
| Carbon monoxide conversion (mole percent) | 70.0 | 58.0 | 57.0 |
| Weight percent $K_2O$ | 0 | 2.0 | 4.0 |
| Percent olefins in $C_2$—$C_4$ fraction | 2.6 | 11.5 | 24.7 |
| BET surface area ($m_2/g$) | 64.0 | 64.0 | ~5.0 |

The results of Runs 1A, 1B, and 1C are summarized above. The poor yield of olefins in the desired $C_2$—$C_4$ range has been improved by the addition of 2 weight percent $K_2O$ to the catalyst of Run 1B. The best yield of olefins in the desired range is obtained in Run 1C.

## Example 2

In Run 2 the catalyst is that of Run 1C. However, in Run 2 the $H_2/CO$ ratio was decreased from 1 to $\frac{1}{2}$. The results of Run 1C and Run 2 are summarized below for comparison.

| | Run 1C | Run 2 |
|---|---|---|
| Paraffins: | | |
| $C_1$ | 37.6 | 24.6 |
| $C_2$ | 25.8 | 23.2 |
| $C_3$ | 13.8 | 15.3 |
| $C_4$ | 4.4 | 5.8 |
| Olefins: | | |
| $C_2$ | 0.9 | 4.2 |
| $C_3$ | 8.7 | 13.8 |
| $C_4$ | 4.8 | 8.0 |
| Olefins & Paraffins: | | |
| $C_5$ | 2.1 | 3.4 |
| $C_6$ | 1.8 | 1.7 |
| Carbon monoxide conversion (mole percent) | 57.0 | 37.0 |
| Weight percent $K_2O$ | 4.0 | 4.0 |
| $H_2/CO$ ratio | 1:1 | 1:2 |
| Percent olefins in $C_2$—$C_4$ fraction | 24.7 | 37.0 |
| BET surface area ($m^2/g$) | ~5.0 | ~5.0 |

These data indicate that a decrease in the $H_2/CO$ ratio increases the yield of olefins in the desired $C_2$ to $C_4$ range at the expense of a drop in the CO conversion.

Example 3

In Run 3, the catalyst was prepared as in Run 1C except that it was composed of 45 percent $MoO_3$, 45 percent $Al_2O_3$, and 10 percent $K_2O$. The results of both Run 1C and Run 3 are summarized below for comparison.

| | Run 1C | Run 3 |
|---|---|---|
| Paraffins: | | |
| $C_1$ | 37.6 | 30.1 |
| $C_2$ | 25.8 | 4.5 |
| $C_3$ | 13.8 | 2.0 |
| $C_4$ | 4.4 | 1.2 |
| Olefins: | | |
| $C_2$ | 0.9 | 6.6 |
| $C_3$ | 8.7 | 6.0 |
| $C_4$ | 4.8 | 2.8 |
| Olefins & paraffins: | | |
| $C_5$ | 2.1 | 0.3 |
| $C_6$ | 1.8 | 2.0 |
| Oil | — | 44.6 |
| Carbon monoxide conversion (mole percent) | 57.0 | 54.0 |
| Weight percent $K_2O$ | 4.0 | 10.0 |
| Percent olefins in $C_2$—$C_4$ fraction | 24.7 | 66.7 |
| BET surface area ($m^2/g$) | ~5.0 | 6.5 |

These data indicate that while an increase in concentration of the second component of this catalyst from 4 percent to 10 percent does not significantly improve the yield of $C_2$—$C_4$ olefins, it does significantly improve the percent of olefins in the $C_2$—$C_4$ hydrocarbon fraction. This increase in the concentration of the second component also changes the olefinic product distribution.

Example 4

For Runs 4A and 4B a commercially available zircon was impregnated via the incipient wetness technique with 10 percent $MoO_3$ to make the catalyst for Run 4A and with 10 percent $MoO_3$ and 5 percent $K_2O$ to make the catalyst for Run 4B. The hourly space velocity in Run 4A of 323 $hr^{-1}$ and the hourly space velocity of Run 4B was 362 $hr^{-1}$.

| | Run 4A | Run 4B |
|---|---|---|
| Paraffins: | | |
| $C_1$ | 62.4 | 19.1 |
| $C_2$ | 25.9 | 6.7 |
| $C_3$ | 7.0 | 5.0 |
| $C_4$ | 1.2 | 2.2 |
| Olefins: | | |
| $C_2$ | — | 1.7 |
| $C_3$ | — | 12.4 |
| $C_4$ | — | 9.0 |
| Olefins & paraffins: | | |
| $C_5$ | — | 1.3 |
| $C_6$ | — | 1.7 |
| Oil | 3.4 | 40.8 |
| Carbon monoxide conversion (mole percent) | 60.0 | 92.0 |
| Weight percent $K_2O$ | 0.0 | 5.0 |
| Percent olefin in $C_2$—$C_4$ fraction | 0.0 | 65.0 |
| BET surface area* ($m^2/g$) | 0.31 | 0.31 |

*Surface area of support only.

These data indicate the dramatic improvement in yield of desired olefins effected by the second component of the catalyst. The presence in Run 4B of the two components, $MoO_3$ and $K_2O$, on a support of low surface area resulted in a high carbon monoxide conversion and a good olefin yield in the $C_2$—$C_4$ fraction.

Example 5

In this run a potassium oxalatomolybdate was prepared by dissolving with stirring 12.7 g (0.1 mole) of oxalic acid and 14.4 g (0.1 mole) of molybdenum trioxide in water to form a solution. After the addition of 12.6 g (0.1 mole) more of oxalic acid and 30.7 g (0.0937 mole) of $K_2MoO_4 \cdot 5H_2O$, a precipitate formed. The pH of the solution, measured at 2, was adjusted to 6 by the addition of a sufficient quantity of 28 percent ammonia. At this point the precipitate disappeared. The solution which had been heated by the addition of ammonia was allowed to cool. Crystals formed, were analyzed by x-ray fluorescence, and were found to contain 25.2 percent Mo, 23.4 percent K, and 5.7 percent C, with the remainder being hydrogen and oxygen. The crystals were dried, placed in a reactor, and hydrogen was passed through the reactor for about a 60-hour period during which time the temperature was raised in steps from 150°C to 520°C with the pressure remaining fixed at $1039 \times 10^2$ Pa (15 psig).

Results:

| | |
|---|---|
| Paraffins: | |
| $C_1$ | 23.8 |
| $C_2$ | 3.9 |
| $C_3$ | 1.4 |
| $C_4$ | 0.9 |
| Olefins: | |
| $C_2$ | 8.3 |
| $C_3$ | 9.5 |
| $C_4$ | 5.8 |
| Olefins & paraffins: | |
| $C_5$ | — |
| $C_6$ & higher | 46.3 |
| Carbon monoxide conversion (mole percent) | 42.7 |
| Percent olefin in $C_2$—$C_4$ fraction | 79.2 |
| BET surface area ($m^2/g$) | 6.1 |

These data illustrate an unsupported catalyst useful in the process of the invention to improve the yield of olefins in the $C_2$ to $C_4$ range.

Example 6

$Al_2O_3$ (105 g) was impregnated with a solution consisting of $MoO_3$ (41.5 g), ethylenediamine tetraacetic acid (42.6 g) and $K_2CO_3$ (8.12 g) dissolved in 500 ml of water with an adjusted (by $NH_4OH$)

pH of about 6. The $Al_2O_3$ was impregnated by dripping the solution onto it while the $Al_2O_3$ was rotated within a heated (120°C) drum. Three individual samples of the resulting catalyst were employed in the following runs.

| | 6A | 6B | 6C |
|---|---|---|---|
| Temperature (°C) | 396 | 396 | 422 |
| Pressure, psig ($kg/cm^2$) | 286 (20.2) | 143 (10.1) | 143 (10.1) |
| HSV ($hr^{-1}$) | 536 | 447 | 422 |
| Paraffins: | | | |
| $C_1$ | 32.0 | 29.9 | 33.6 |
| $C_2$ | 5.4 | 4.1 | 5.3 |
| $C_3$ | 3.7 | 5.0 | 6.1 |
| $C_4$ | 1.3 | 1.2 | 1.4 |
| Olefins: | | | |
| $C_2$ | 9.2 | 11.0 | 11.2 |
| $C_3$ | 3.7 | 5.4 | 6.4 |
| $C_4$ | 2.1 | 2.2 | 2.4 |
| Olefin & paraffin: | | | |
| $C_5$ & higher | 41.3 | 41.1 | 33.7 |
| Carbon monoxide conversion (mole percent) | 48.4 | 32.0 | 45.9 |
| Percent olefin in $C_2$—$C_4$ fraction | 59.0 | 64.0 | 61.0 |
| BET surface area* ($m^2/g$) | 4.0 | 4.0 | 4.0 |

*Surface area of support only.

A comparison of the 6A and 6B results demonstrate that a reduction of pressure increases the selectivity of $C_2$—$C_4$ olefins but at the expense of carbon monoxide conversion. However, the results of 6C demonstrate that this increased selectivity can be maintained without substantial sacrifice of carbon monoxide conversion by an increase in temperature.

Example 7

To demonstrate the sulfur tolerance of certain catalyst species of this invention, particularly those comprising a sulfide, oxide or metal of molybdenum, a catalyst comprising 10 percent $MoO_3$, 2 percent $K_2O$ and 88 percent Carborundum SAEHS-33 $Al_2O_3$ was first exposed to the following conditions for 366 hours:

| | |
|---|---|
| Temperature (°C) | 417 |
| Pressure (psig) | 287 (20.2 $kg/cm^2$) |
| HSV ($hr^{-1}$) | 282 |
| $H_2/CO$ | 0.94 |
| BET surface area* ($m^2/g$) | 4 |

*Surface area of support only.

After this first exposure, the catalyst was then exposed to an additional 116 hours of the same conditions except that 20 ppm $H_2S$ was added and the HSV increased to 356 $hr^{-1}$. The results of a gas phase analysis of both exposures are reported below:

| | Without $H_2S$ | With $H_2S$ |
|---|---|---|
| Paraffins: | | |
| $C_1$ | 39.8 | 43.0 |
| $C_2$ | 7.2 | 6.2 |
| $C_3$ | 8.4 | 2.2 |
| $C_4$ | 1.0 | 1.1 |
| Olefins: | | |
| $C_2$ | 22.7 | 19.8 |
| $C_3$ | 16.6 | 19.9 |
| $C_4$ | 4.3 | 6.9 |
| Carbon monoxide conversion (mole percent) | 41.7 | 37.1 |

These data demonstrate no significant change in activity (carbon monoxide conversion) or gas phase olefin selectivity upon exposure to a relatively large concentration of $H_2S$.

Example 8 and Control

Two samples each of equal weight of catalyst Mo-1201 (surface area of 160 $m^2/g$), and Mo-0502 (surface area of 60 $m^2/g$), both 10 percent molybdenum trioxide on alumina were used as catalysts in these experiments. One sample of each catalyst was alkalized with potassium carbonate while the other sample of each catalyst was alkalized in a similar fashion plus sulfided with potassium sulfide, ammonium sulfide and molybdenum sulfide. All the catalysts were then reduced with hydrogen at 500°C and subsequently used to catalyze a Fischer-Tropsch hydrocarbon synthesis. The parameters and results of these experiments are reported below:

| | Mo-1201 | Mo-0502 |
|---|---|---|
| Process parameters: | | |
| Catalyst surface area ($m^2/g$) | 160 | 60 |
| Pressure, psig ($kg/cm^2$) | 300 (21.2) | 300 (21.2) |
| $H_2/CO$ ratio | 0.8—1 | 0.8—1 |
| Temperature (°C) | | |
| A. | 400 | 400 |
| B. | 370 | 370 |
| Process results: | | |
| (% hydrocarbon product) | | |
| A. Oxide form of catalyst | | |
| (10% $MoO_3$, 2% $K_2O$) | | |
| $C_1$ | 47 | 43 |
| $C_2$—$C_4$ (saturated) | 51 | 51 |
| $C_2$—$C_4$ (unsaturated) | 1 | 5 |
| $C_5$+ | 1 | 1 |
| Total | 100 | 100 |
| B. Sulfide form of catalyst | | |
| (15% $MoO_3$, 2.7% $K_2O$) | | |
| $C_1$ | 60 | 54 |
| $C_2$—$C_4$ (saturated) | 40 | 43 |
| $C_2$—$C_4$ (unsaturated) | 0 | 3 |
| $C_5$+ | 0 | 0 |
| Total | 100 | 100 |

The above data demonstrates the surprising effect that surface area has on the synthesis of $C_2$—$C_4$ olefinic hydrocarbons, specifically:

(a) the oxide form of catalyst Mo-1201 having a surface area of 160 $m^2/g$ produced only 1 percent $C_2$—$C_4$ olefinic hydrocarbon and the sulfide form of the same catalyst produced no $C_2$—$C_4$ olefinic hydrocarbon; and

(b) the oxide form of catalyst Mo-0502 having a surface area of 60 $m^2/g$ produced 5 percent $C_2$—$C_4$ olefinic hydrocarbon and the sulfide form of the same catalyst produced 3 percent $C_2$—$C_4$ olefinic hydrocarbon.

**Claims**

1. A process for producing olefins $C_2$—$C_4$ hydrocarbons by contacting carbon monoxide and hydrogen at reactive conditions, characterized by contacting the carbon monoxide and hydrogen with a catalyst having a surface area less than 100 $m^2/g$ and consisting of:

(A) between 1 percent and 95 percent by weight based upon the weight of the catalyst of at least one material selected from the group consisting of the sulfide, oxide or metal of molybdenum, tungsten, rhenium, ruthenium, nickel, palladium, rhodium, osmium, iridium and platinum; and

(B) between 0.05 percent and 50 percent by weight based upon the weight of the catalyst of at least one material selected from the group consisting of the hydroxide, oxide or salt of lithium, sodium, potassium, rubidium, cesium, magnesium, calcium, strontium, barium and thorium.

2. The process of Claim 1 wherein the catalyst has a surface area less than 40 $m^2/g$.

3. The process of Claim 2 wherein the catalyst consists essentially of, in addition to A and B, between 1 and 98.95 percent by weight, based upon the weight of the catalyst, of a support.

4. The process of Claim 3 wherein the support is alumina, carbon, silica, zirconia, zircon, magnesia, titanium dioxide or mixtures thereof.

5. The process of Claim 3 wherein B is at least one material selected from the group consisting of the hydroxide, oxide or salt of lithium, sodium, potassium, rubidium or cesium.

6. The process of Claim 3 wherein A is at least one material selected from the group consisting of the sulfide, oxide or metal of molybdenum or tungsten.

7. The process of Claim 3 wherein the catalyst consists essentially of between 10 and 50 percent A.

8. The process of Claim 7 wherein the catalyst consists essentially of between 1 and 10 percent B.

9. The process of Claim 8 wherein the catalyst consists essentially of at least 40 percent of the support.

**Revendications**

1. Procédé pour la fabrication d'hydrocarbures par contact de l'oxyde de carbone et de l'hydrogène dans des conditions réactives, caractérisé par la mise en contact de l'oxyde de carbone et de l'hydrogène avec un catalyseur ayant une surface spécifique inférieure à 100 $m^2/g$ et constitué par:

(A) entre 1% et 95% en poids, calculé sur le poids du catalyseur, d'au moins une matière choisie parmi le groupe constitué par le sulfure, l'oxyde ou le métal de: molybdène, tungstène, rhénium, ruthénium, nickel, palladium, rhodium, osmium, iridium et platine; et

(B) entre 0,05 % et 50 % en poids, calculé sur le poids du catalyseur, d'au moins une matière choisie parmi le groupe constitué par l'hydroxyde, l'oxyde ou le sel de: lithium, sodium, potassium, rubidium, césium, magnésium, calcium, strontium, baryum et thorium.

2. Procédé selon la revendication 1, dans lequel le catalyseur a une surface spécifique inférieure à 40 $m^2/g$.

3. Procédé selon la revendication 2, dans lequel le catalyseur est constitué essentiellement par l'addition à A et à B, entre 1 et 98,95 % en poids d'un support, calculé sur le poids du catalyseur.

4. Procédé selon la revendication 3, dans lequel le support est l'alumine, le carbone, la silice, la zircone, le zircon, la magnésie, le dioxyde de titane et leurs mélanges.

5. Procédé selon la revendication 3, dans lequel B est au moins une matière choisie parmi le groupe constitué par l'hydroxyde, l'oxyde ou le sel de lithum, sodium, potassium, rubidium ou césium.

6. Procédé selon la revendication 3, dans lequel A est au moins une matière choisie parmi le groupe constitué par le sulfure, l'oxyde ou le métal de nolybdène ou de tungstène.

7. Procédé selon la revendication 3, dans lequel le catalyseur est constitué essentiellement de 10 à 50 % de A.

8. Procédé selon la revendication 7, dans lequel le catalyseur est constitué essentiellement entre 1 et 10 % de B.

9. Procédé selon la revendication 8, dans lequel le catalyseur est constitué essentiellement par au moins 40 % du support.

**Patentansprüche**

1. Verfahren zum Erzeugen gesättigter und ungesättigter, zwei bis vier Kohlenstoffatome enthaltender Kohlenwasserstoffe, indem man Kohlenmonoxid und Wasserstoff unter Reaktionsbedingungen in Berührung bringt, dadurch gekennzeichnet, dass das Kohlenmonoxid und der Wasserstoff mit einem Katalysator in Berührung gebracht werden, der besteht aus:

(A) Zwischen 1 und 95 Gewichtsprozent—gerechnet auf das Katalysatorgewicht—wenigstens eines Stoffes, der aus der folgenden Gruppe ausgewählt wird: Molybdän-, Wolfram-, Rhenium-, Ruthenium- oder Platinmetall oder -oxid oder -sulfid;

(B) Zwischen 0,05 und 50 Gewichtsprozent—gerechnet auf das Katalysatorgewicht—wenigstens eines Stoffes, der aus der folgenden Gruppe ausgewählt wird: Lithium, Natrium-, Kalium-, Rubidium-, Cäsium-, Magnesium-, Calcium-, Strontium-, Barium- und Thoriumhyroxid, -oxid oder -salz; und

(C) Wenigstens 1 Gewichtsprozent—gerechnet auf das Katalysatorgewicht—eines Trägers.

2. Verfahren gemäss Anspruch 1, worin C Kohlenstoff, Tonerde, Kieselerde, Zirconerde, Magnesia oder Gemische derselben bedeutet.

3. Verfahren gemäss Anspruch 1, worin B mindestens ein Stoff aus der folgenden Gruppe ist: Lithium-, Natrium-, Kalium-, Rubidium- und Cäsiumhydroxid, -oxid oder -salz.

4. Verfahren gemäss Anspruch 1, worin A mindestens ein Stoff aus der folgenden Gruppe ist: Molybdän- oder Wolframsulfid, -oxid oder -metall.

5. Verfahren gemäss Anspruch 1, worin C in einer Konzentration von 50 bis 80 Gewichtsprozent—gerechnet auf das Katalysatorgewicht—vorliegt.

6. Verfahren gemäss Anspruch 3, wobei A mindestens ein Stoff aus der Gruppe metallisches Molybdän oder Wolfram oder -sulfid oder -oxid ist.

7. Verfahren gemäss Anspruch 3, wobei der Katalysator im wesentlichen aus 10 bis 50 Prozent A besteht.

8. Verfahren gemäss Anspruch 7, wobei der Katalysator im wesentlichen aus 1 bis 10 Prozent B besteht.

9. Verfahren gemäss Anspruch 8, wobei der Katalysator im wesentlichen aus mindestens 40 Prozent Träger besteht.